# EUROPÄISCHE PATENTSCHRIFT

(11) **EP 1 066 111 B1**
(45) Veröffentlichungstag und Bekanntmachung des Hinweises auf die Patenterteilung: **20.08.2003**
(21) Anmeldenummer: 98962366.5
(22) Anmeldetag: 20.11.1998
(51) Int. Cl.: B01J 25/02, B01J 37/00, C07C 209/36

(54) **RANEY-NICKEL-KATALYSATOREN, EIN VERFAHREN ZU DEREN HERSTELLUNG UND DEREN VERWENDUNG ZUR HYDRIERUNG ORGANISCHER VERBINDUNGEN**
RANEY NICKEL CATALYSTS, A METHOD FOR PRODUCING SAID CATALYSTS AND THE USE THEREOF FOR HYDROGENATING ORGANIC COMPOUNDS
CATALYSEURS AU NICKEL DE RANEY, LEUR PROCEDE DE PREPARATION ET LEUR UTILISATION POUR HYDROGENER DES COMPOSES ORGANIQUES

(30) Priorität: 03.12.1997 DE 19753501
(43) Veröffentlichungstag der Anmeldung: 10.01.2001
(73) Patentinhaber: BAYER AG, 51368 Leverkusen (DE)
(72) Erfinder: WEGENER, Gerhard, D-40822 Mettmann (DE); WALDAU, Eckart, D-40597 Düsseldorf (DE); PENNEMANN, Bernd, D-51061 Köln (DE); TEMME, Bodo, D-41540 Dormagen (DE); WARLIMONT, Hans, D-01326 Dresden (DE); KÜHN, Uta, D-01728 Possendorf-Börnchen (DE)
(86) Internationale Anmeldenummer: EP9807457
(87) Internationale Veröffentlichungsnummer: WO99028028

(56) Entgegenhaltungen:
- EP-A- 0 437 788
- DE-A- 3 537 247
- US-A- 3 839 011

## Beschreibung

Die Erfindung betrifft Raney-Nickel-Katalysatoren mit verbesserter Standzeit, ein Verfahren zu deren Herstellung und deren Verwendung bei der Hydrierung organischer Verbindungen, insbesondere bei der Hydrierung aromatischer Nitroverbindungen.

Die Herstellung und Anwendung von RaNi als Hydrierkatalysator von aromatischen Nitroverbindungen wie z.B. Nitrobenzol, Nitrotoluolen, Dinitrotoluolen, chlorierten Nitroaromaten und anderen ist bekannt und öfters beschrieben worden (z.B. R Schröter, Angew. Chem. 1941, 54, 229, EP-A 0223035). Üblicherweise geht man bei der Herstellung von RaNi Katalysatoren von einer Legierung aus Aluminium mit Nickel und gegebenenfalls einem oder mehreren weiteren Nebengruppenmetallen aus. Die Legierung wird beispielsweise durch Schmelzen oder Reaktivmahlen der Ausgangsmetalle erhalten. RaNi Katalysatoren lassen sich durch Legieren der Ausgangslegierung mit andereren Metallen zur hinsichtlich Aktivität, Selektivität und Stabilität insbesondere bei erhöhten Temperaturen modifizieren. Diese Dotierung des Katalysatores durch Zusatz verschiedenster Metalle zur Al-Ni Schmelze der Katalysatorvorläufer ist bekannt (DE-A 40 01 484, DE-A 35 37 247). Die Katalysatorvorläufer werden durch Verdüsung der Al-Ni-Metallschmelze oder mechanisch zerkleinert und der Katalysator wird durch teilweise oder vollständige Auslaugung des Aluminiums aus der Legierung mit einer Base freigesetzt (DE-A 27 13 374). Die katalytische Wirkung der aus den Legierungen hervorgehenden Katalysatoren hängt u.a. von der qualitativen und quantitativen Zusammensetzung der Legierung, der Struktur und dem Gefüge der Legierung und damit von der resultierenden Struktur und dem resultierenden Gefüge des Katalysators ab.

Die Hydrierung von aromatischen Nitroverbindungen ist eine großtechnisch häufig durchgeführte Reaktion. Hierfür werden vielfach RaNi Katalysatoren eingesetzt. Kataivsatorstandzeiten, Strukturen und Gefuge der Ausgangslegierungen sowie die Erstarrungsgeschwindigkeit sind kaum korreliert. Insbesondere bei den ternären Sytemen Al-Ni-Zusatzmetall kann in der Ausgangslegierung eine Vielzahl von Phasen vorliegen, die im resultierenden Katalysator keine oder nur geringe Aktivitäten bzw. hohe Katalysatorverbräuche aufweisen. Aktivitäten und Standzeiten unterschiedlicher Katalysatorchargen werden damit schwer reproduzierbar.

Es bestand daher die Aufgabe, RaNi-Katalysatoren zur Verfügung zu stellen, die bei der Hydrierung aromatischer Nitroverbindungen eine verbesserte Standzeit und damit geringeren Katalysatorverbrauch aufweisen.

Es wurde jetzt gefunden, daß sich die Standzeit von RaNi Katalysatoren in der Hydrierung der Nitrogruppen von aromatischen Nitroverbindungen gegenüber den üblicherweise eingesetzten Katalysatoren überraschenderweise erhöht, wenn erfindungsgemäß amorphe, teilamorphe oder feinkristalline durch Rascherstarrung hergestellte Legierungen als Vorläufer für die Katalysatoren eingesetzt werden.

Gegenstand der Erfindung sind somit RaNi-Katalysatoren, welche dadurch erhalten werden, daß man die Schmelze einer Legierung, enthaltend 50 bis 95 Gew.-% Aluminium, 10 bis 50 Gew.-% Nickel, sowie gegebenenfalls 0 bis 20 Gew.-% Eisen, 0 bis 15 Gew.-% Cer, Cer-Mischmetall, Vanadium, Niob, Tantal, Chrom, Molybdän oder Mangan sowie gegebenenfalls weiteren glasbildenden Elementen mit einer Abkühlrate > 10⁴K/s rasch erstarren läßt und die rasch erstarrte Legierung anschließend einer Behandlung mit organischen oder anorganischen Basen unterzieht. Erfindungsgemäß erfolgt die Rascherstarrung durch Ausdrücken der Legierungsschmelze auf ein rotierendes Kühlrad oder in den Spalt zwischen zwei im Gegensinn rotierenden Kühlrädern oder durch Schmelzextraktion.

Durch das Abschrecken einer metallischen Schmelze mit der angegebenen Abkühlrate lassen sich metastabile Phasen und Strukturen außerhalb des Gleichgewichtszustandes erhalten und einfrieren. Eine Verfeinerung des Gefüges, d.h. kleinere Kristallitgrößen, im Bereich von 1 - 10 µm und darunter, bevorzugt < 2 µm, werden so bei den erfindungsgemäßen Katalysatoren eingestellt. Unter Rascherstarrung wird hier Abschreckung mit einer Abkühlrate ≥ 10⁴ K/s verstanden, während die Abkühlraten bei der normalen, nicht erfindungsgemäßen Verdüsung zwischen 10² und 10³ K/s liegen. Erfolgt die Rascherstarrung der Legierungsschmelze mit > 10⁴ K/s, so werden amorphe Legierungen oder Legierungen mit kristallinen und amorphen Bereichen, im weiteren als teilamorph bezeichnet, oder vollständig feinkristalline Zustände erhalten. Mit dem Begriff amorph im Zusammenhang mit metallischen Phasen, auch als metallische Gläser oder unterkühlte, feste Schmelzen klassifiziert, wird das Fehlen von Kristallinität beschrieben. Überraschend wurde gefunden, daß die erfindungsgemäß hergestellten RaNi Katalysatoren wesentlich standzeitstabiler sind als solche aus nicht rascherstarrten Legierungen.

Die für die Rascherstarrung erforderlichen hohen Abkühlgeschwindigkeiten von 10⁴ bis 10⁷ K/s lassen sich z.B. durch Ausdrücken einer Legierungsschmelze auf ein rotierendes Kühlrad erzielen (R. W. Cahn, P. Haasen, E. J. Kramer, Materials Science and Technology Vol 8, 1996, p. 237). Rascherstarrte Legierungen können auch mit dem Schmelzextraktionsverfahren hergestellt werden.

Die Ausbildung amorpher Strukturen kann zusätzlich durch Beifügung von weiteren Legierungsmetallen positiv beeinflußt werden. Als derartige Legierungsmetalle im erfindungsgemäßen Katalysator werden Seltenerd-Metalle, bevorzugt Cer oder Cer-Mischmetall, und/oder ausgewählte Nebengruppenelemente, bevorzugt Vanadium, Niob, Tantal, Chrom, Molybdän oder Mangan eingesetzt. Gegebenenfalls können noch weitere glasbildende Hauptgruppenelemente, bevorzugt Bor, Silicium, Kohlenstoff und/oder Phosphor, enthalten sein.

Die durch Rascherstarrung bewirkte Verbesserung der katalytischen Eigenschaften wird durch Temperung der Legierungen zerstört, da sich bei der Temperung wieder die Gleichgewichtszustände einstellen. Der Anteil an amorpher Phase in einer Legierung läßt sich mittels Röntgendiffraktometrie und durch metallographische Untersuchungen von Legierungsschliffen am Lichtmikroskop nachweisen (A. Molnar et al., Adv. Catal., 1989, 36, 329).

Die erfindungsgemäßen Katalysatoren auf Basis amorpher/teilamorpher oder feinkristalliner durch Rascherstarrung hergestellter Legierungen zeichnen sich durch erhöhte Standzeiten und geringe Nebenproduktbildung, sowie durch bessere Reproduzierbarkeit der Katalysatoreigenschaften im Vergleich zu herkömmlichen Katalysatoren aus. Der Katalysatorbedarf im Einsatz im großtechnischen Maßstab wird dadurch reduziert. Dies kommt z.B. bei der technischen Herstellung von 2,4-/2,6-Tolylendiamin durch Hydrierung von Dinitrotoluolen oder anderen aromatischen Nitroverbindungen insbesondere in der fremdlösungsmittelfreien Hydrierung vorteilhaft zur Auswirkung.

Ein weiterer Gegenstand der Erfindung ist ein Verfahren zur Herstellung von RaNi-Katalysatoren, gemäß dem Gegenstand der Ansprüche 1 bzw. 2

Für die erfindungsgemäße Herstellung der Katalysatoren aus rasch erstarrten Legierungen bzw. für die Herstellung amorpher, teilamorpher oder feinkristalliner Legierungen als Katalysatorvorläufer werden Legierungen enthaltend 50 bis 95 Gew% Aluminium, 10 bis 50 Gew% Nickel, gegebenenfalls 0 bis 20 Gew% Eisen sowie 0 bis 15 Gew% Cer, Cer-Mischmetall, Vanadium, Niob, Tantal, Chrom, Molybdän oder Mangan, eingesetzt. Bevorzugt sind Legierungen aus 60 bis 90 Gew% Aluminium, 15 bis 40 Gew% Nickel, 0 bis 10 Gew% Eisen und/oder anderen Übergangsmetallen sowie 0 bis 10 Gew% Cer, Cer-Mischmetall, Vanadium, Niob, Tantal, Chrom, Molybdän oder Mangan. Besonders bevorzugt sind Legierungen aus 70 bis 85 Gew% Aluminium, 15 bis 30 Gew% Nickel, 0 bis 6 Gew% Eisen und/oder anderen Übergangsmetallen sowie 0 bis 10 Gew% Cer, Cer-Mischmetall, Vanadium, Niob, Tantal, Chrom, Molybdän oder Mangan.

Diese Legierungen können z. B. durch induktives Erschmelzen der Metalle in entsprechenden Gewichtsverhältnissen hergestellt werden.

Die für die Rascherstarrung der zuvor beschriebenen Legierungen erforderlichen hohen Abkühlgeschwindigkeiten lassen sich durch Ausdrücken der Legierungsschmelze auf ein rotierendes Kühlrad oder in den Spalt zwischen zwei im Gegensinn rotierenden Kühlrädern, durch das Schmelzextraktionsverfahren erzielen. Die Dicke der auf Kühlrädern erhaltenen Bänder liegt zwischen 10 und 100 µm, bevorzugt zwischen 10 und 50 µm. Das Kühlrad besteht bevorzugt aus Cu, Ag oder Cuund Ag-Basislegierungen, kann aber auch aus beliebigen anderen metallischen Werkstoffen hergestellt werden. Kühlung der Kühlwalze ist möglich mit Raumluft, gekühlten und kondensierten Gasen, durch Wasser oder ein beliebiges anderes Kühlmedium.

Die Freisetzung der RaNi-Katalysatoren wird durch Alkalibehandlung der gegebenenfalls zerkleinerten rasch erstarrten Legierung mit wäßrigen Lösungen von organischen oder anorganischen Basen, beispielsweise mit Natrium- oder Kaliumhydroxid, Natrium- oder Kaliumcarbonat, bevorzugt mit Natrium- oder Kaliumhydroxid, vorzugsweise bei Temperaturen von 50 bis 100°C durchgeführt. Die Menge an Base richtet sich nach der Menge des in der Legierung vorliegenden Aluminiums. Die Base kann stöchiometrisch, im Überschuß oder Unterschuß zum Aluminium eingesetzt werden. Bevorzugt ist ein Mengenverhältnis von Aluminium zu Base von 1:1 bis 1:10, besonders bevorzugt ein Verhältnis von 1:1.1 bis 1:5. Der Katalysator kann durch teilweises oder vollständiges Dekantieren oder Abfiltrieren der wäßrigen Lösung isoliert werden und durch wiederholtes Dekantieren oder Abfiltrieren von zugefügter Waschlösung gewaschen werden Als Waschlösung wird Wasser (entionisiert, destilliert oder Trinkwasser) oder eine Lösung von Natrium- oder Kaliumhydroxid in Wasser verwendet.

Die durch Auslaugung aus den beschriebenen rasch erstarrten amorphen, teilamorphen oder feinkristallinen Legierungen herstellbaren Katalysatoren enthalten Restmengen von 0 bis 15 Gew% Aluminium, 50 bis 100 Gew% Nickel, 0 bis 50 Gew% Eisen und/oder andere Übergangsmetalle sowie 0 bis 30 Gew% Cer, Cer-Mischmetall, Vanadium, Niob, Tantal, Chrom, Molybdän oder Mangan. Bevorzugt sind Katalysatoren aus 0 bis 10 Gew% Aluminium, 60 bis 100 Gew% Nickel, 0 bis 30 Gew% Eisen und/oder anderen Übergangsmetallen sowie 0 bis 30 Gew% Cer, Cer-Mischmetall, Vanadium, Niob, Tantal, Chrom, Molybdän oder Mangan. Besonders bevorzugt sind Legierungen aus 0 bis 10 Gew% Aluminium, 75 bis 100 Gew% Nickel, 0 bis 20 Gew% Eisen und/oder anderen Übergangsmetallen sowie 0 bis 25 Gew% Cer, Cer-Mischmetall, Vanadium, Niob, Tantal, Chrom, Molybdän oder Mangan.

Ein weiterer Gegenstand der Erfindung ist die Verwendung der beschriebenen Katalysatoren zur Hydrierung von organischen Verbindungen, bevorzugt zur Hydrierung aromatischer Nitroverbindungen.

Als Ausgangsmaterialien eignen sich dabei beliebige aromatische Nitroverbindungen, z.B. Nitrobenzol, die Isomeren und deren Gemische von Nitrotoluol, chlorierten Nitroaromaten, Dinitronaphtalin und insbesondere die Isomeren und Isomerengemische des Dinitrotoluols. Die Nitroverbindungen werden vorzugsweise in Substanz, also ohne Verwendung eines Fremdlösungsmittels, bei Temperaturen von 120 bis 250°C, vorzugsweise von 140°C bis 200°C, und einem Druck von 5 bis 50 bar, vorzugsweise bei 10 bis 30 bar, in Gegenwart des im Reaktionsmedium suspendierten Katalysators hydriert. Das Reaktionsmedium setzt sich aus Produkt, entsprechend gebildetem Wasser und der Gasphase zusammen.

Dem Reaktionsmedium kann dabei gegebenenfalls auch ein Lösungsmittel, wie z.B. ein Alkohol, bevorzugt Methanol oder 2-Propanol, zugesetzt werden. Die Hydrierung anderer Nitroverbindungen erfolgt oft in einem Lösungsmittel, wie z.B. in einem Alkohol, bevorzugt in Methanol oder 2-Propanol, bei einem Druck von 5 bis 200 bar.

Die Hydrierung kann kontinuierlich oder diskontinuierlich in den üblichen Reaktoren erfolgen. Die Menge der in den Reaktor eingespeisten Nitroverbindung entspricht dabei der gleichzeitig aus dem Reaktor ausgeschleusten Menge an Reaktionsprodukt.

Überraschend und nicht voraussehbar ist die Erhöhung der Standzeit der erfindungsgemäßen Katalysatoren im Vergleich zu Katalysatoren aus konventionell hergestellten Vorlegierungen. Die beobachtete Steigerung der Standzeit der erfindungsgemäßen Katalysatoren kann deutlich an Beispielen der fremdlösungsmittelfreien Reduktion von technischen 2,4/2,6-Dinitrotoluol Gemischen demonstriert werden.

### Beispiele

### Herstellung von Legierungen

### Beispiel 1 (Vergleich) Al80Ni20 durch Verdüsung

160 kg Aluminium und 40 kg Nickel wurden im Induktionsofen geschmolzen, auf 1300°C erhitzt und dann in einen beheizten Keramikgießtrichter gegossen. Der aus dem mittigen Bodenauslauf austretende flüssige Metallstrahl von 15 mm Durchmesser wurde mit 6.2 bar Druckluft, durch eine konzentrisch angeordnete Ringspaltdüse eingeblasen, zerstäubt. Das verdüste Pulver wurde in einem Wassertank aufgefangen. Das Pulver wurde isoliert und im warmen Luftstrom getrocknet.

### Beispiel 2 Al80Ni20 durch Rascherstarrung

Die Vorlegierung wurde aus 800 g Aluminium und 200 g Nickel im Vakuuminduktionsofen im Aluminiumoxid-Tiegel erschmolzen und in eine Kupferkokille abgegossen. Von diesen in Stangenform vorgeschmolzenen Ingots wurden ca. 15 g in den Tiegel mit angeschmolzener Düse einer Schmelzspinnanlage eingesetzt. Das induktive Aufschmelzen der Vorlegierung erfolgt in ca. 2 min mit einer Überhitzung von ca. 150°C. Die Schmelze wurde mittels Argon durch die Düse auf ein rotierendes Kupfer-Kühlrad mit einer mittleren Banddicke von 20 µm ausgepreßt.

### Beispiel 3 Al80Ni17Fe3 durch Rascherstarrung

Die rasch erstarrte Legierung wurde analog zu Beispiel 2 aus 800 g Aluminium, 170 g Nickel und 30 g Eisen hergestellt.

### Beispiel 4 Temperung von Al80Ni17Fe3 aus Beispiel 3

200 g einer nach Beispiel 3 hergestellten rascherstarrten Al80Ni17Fe3 Legierung wurden eine Stunde bei 600°C unter Argon getempert.

### Beispiel 5 und 6 Teilamorphes und feinkristallines Al79Ni17Fe3Ce1 durch Rascherstarrung

Die rasch erstarrte Legierung wurde analog Beispiel 2 aus 790 g Aluminium, 170 g Nickel, 30 g Eisen und 10 g Cer hergestellt. Aus dem erhaltenem Material wurde jeder Abguß (jeweils ca. 15 g) röntgenographisch untersucht. Bänder mit nachgewiesenem amorphen Anteil wurden in der teilamorphen Charge (Beispiel 5) zusammengefaßt (ca. 50%), Bänder, bei denen röntgenographisch keine amorphen Anteile nachgewiesen werden konnten, wurden in der feinkristallinen Charge (Beispiel 6) zusammengefaßt.

### Herstellung eines Katalysators

782 g Natriumhydroxid wurden in 3129 g Wasser gelöst und die Temperatur der erhaltenen Natronlauge auf 80°C eingestellt. Unter Stickstoffüberlagerung wurden 200 g der zerkleinerten pulverförmigen Ausgangslegierung so zu der Natronlauge gegeben, daß die Temperatur bei 80±2°C gehalten wurde und die Schaumbildung nicht zu stark wurde. Das Reaktionsgemisch wurde 30 Minuten bei 80°C nachgerührt. Im Anschluß wurde die überstehende Lauge abdekantiert und der Rückstand mit einer Lösung von 78 g Natriumhydroxid in 313 g Wasser 5 Minuten unter Rühren nachbehandelt. Auch diese Lauge wurde dekantiert und der Katalysator wurde mit Wasser bis zu einem pH-Wert von 8 bis 9 gewaschen. Der Katalysator wurde quantitativ als wäßriger Schlamm erhalten.

### Hydrierung von DNT

Verwendet wurde ein Autoklav mit 160 ml Volumen, ausgestattet mit einem Begasungsrührer, einer Wasserstoffzuführungsleitung, einem Einleitungsrohr für die Nitroverbindung und einem Auslaßventil für überschüssigen Wasserstoff. Das Reaktionsgemisch verließ den Reaktor durch eine Fritte, die den Katalysator zurückhielt. Die Temperatur im Reaktor wurde durch einen äußeren Heiz- bzw. Kühlkreislauf geregelt. Eine Kühlschlange im Inneren des Reaktors sorgte für zusätzliche Kühlung des Reaktionsgemisches. 180 g eines Gemisches aus einem Gemisch aus 80% 2,4-Diaminotoluol und 20% 2,6-Diaminotoluol (TDA) mit Wasser im Gewichtsverhältnis von TDA zu Wasser von 63:37 sowie 4 g Katalysator wurden im Reaktor vorgelegt. Anschließend wurde der Reaktorinhalt mit Wasserstoff unter Druck gesetzt und aufgeheizt. Bei einer Temperatur von 180°C und einem Druck von 26 bar wurden stündlich 109 g eines Gemisches aus 80% 2,4-Dinitrotoluol und 20% 2,6-Dinitrotoluol in den Reaktor geleitet und bis zur Erschöpfung des Katalysators hydriert. Das resultierende Hydrierungsprodukt wurde kontinuierlich aus dem Reaktor entnommen und zum reinen Diaminotoluol Isomerengemisch aufgearbeitet.

### Zusammenfassung der Ergebnisse

| Beispiel | Legierung | Struktur / Herstellung | TDA-Ausbeute % d. Theorie | Standzeit h |
|---|---|---|---|---|
| 1 (Vergleich) | Al80Ni20 | Kristallin / Luft in Wasser Verdüsung | 97.1 | 18 |
| 2 | Al80Ni20 | Feinkristallin / Rascherstarrung auf Kühlwalze | 98.0 | 115 |
| 3 | Al80Ni17Fe3 | Feinkristallin / Rascherstarrung auf Kühlwalze | 97.6 | 128 |
| 4 | Al80Ni17Fe3 | Grobkristallin / Temperung von Beispiel 3 | 98.0 | 54 |
| 5 | Al79Ni17Fe3Ce1 | Teilamorph / Rascherstarrung auf Kühlwalze | 98.0 | 215 |
| 6 | Al79Ni17Fe3Ce1 | Feinkristallin / Sortierung aus Beispiel 5 | 97.8 | 94 |

## Patentansprüche

1. Verfahren zur Herstellung von Raney-Nickel-Katalysatoren, **dadurch gekennzeichnet, dass** man die Schmelze einer Legierung aus 50 bis 95 Gew.-% Aluminium, 10 bis 50 Gew.-% Nickel, 0 bis 20 Gew.-% Eisen, 0 bis 15 Gew.-% Cer, Cer-Mischmetall, Vanadium, Niob, Tantal, Chrom, Molybdän oder Mangan sowie gegebenenfalls auch weiteren glasbildenden Elementen mit einer Abkühlrate >10⁴ K/s durch Ausdrücken der Legierungsschmelze auf ein rotierendes Kühlrad oder in den Spalt zwischen zwei im Gegensinn rotierenden Kühlrädern abkühlen lässt und anschließend die rasch erstarrte Legierung einer Behandlung mit organischen oder anorganischen Basen unterzieht.

2. Verfahren zur Herstellung von Raney-Nickel-Katalysatoren, **dadurch gekennzeichnet, dass** man die Schmelze einer Legierung aus 50 bis 95 Gew.-% Aluminium, 10 bis 50 Gew.-% Nickel, 0 bis 20 Gew.-% Eisen, 0 bis 15 Gew.-% Cer, Cer-Mischmetall, Vanadium, Niob, Tantal, Chrom, Molybdän oder Mangan sowie gegebenenfalls auch weiteren glasbildenden Elementen mit einer Abkühlrate >10⁴ K/s durch Schmelzextration abkühlen lässt und anschließend die rasch erstarrte Legierung einer Behandlung mit organischen oder anorganischen Basen unterzieht.

3. Raney-Nickel-Katalysatoren erhältlich nach dem Verfahren nach Anspruch 1.

4. Raney-Nickel-Katalysatoren erhältlich nach dem Verfahren nach Anspruch 2.

5. Raney-Nickel-Katalysatoren nach Anspruch 3 oder 4, **dadurch gekennzeichnet, dass** sie als weitere glasbildende Elemente Bor, Silicium, Kohlenstoff und/oder Phophor in einer Menge von 0 bis 50 Gew.-% enthalten.

6. Verwendung eines Raney-Nickel-Katalysators gemäß einem der Ansprüche 3 bis 5 zur Hydrierung von organischen Verbindungen.

7. Verwendung gemäß Anspruch 6 zur Hydrierung aromatischer Nitroverbindungen.

8. Verwendung gemäß Anspruch 6 zur Hydrierung von 2,4-/2,6-Dinitrotoluolgemischen.

## Claims

1. Process for the production of Raney nickel catalysts, **characterised in that** the melt of an alloy of 50 to 95 wt.% of aluminium, 10 to 50 wt.% of nickel, 0 to 20 wt.% of iron, 0 to 15 wt.% of cerium, cerium mixed metal, vanadium, niobium, tantalum, chromium, molybdenum or manganese as well as optionally also further glass-forming elements is cooled at a cooling rate of > 10⁴ K/sec by forcing out the alloy melt onto a rotating cooling wheel or into the gap between two cooling wheels rotating in opposite directions and the rapidly solidified alloy is then subjected to a treatment with organic or inorganic bases.

2. Process for the production of Raney nickel catalysts, **characterised in that** the melt of an alloy of 50 to 95 wt.% of aluminium, 10 to 50 wt.% of nickel, 0 to 20 wt.% of iron, 0 to 15 wt.% of cerium, cerium mixed metal, vanadium, niobium, tantalum, chromium, molybdenum or manganese as well as optionally also further glass-forming elements is cooled at a cooling rate of > 10⁴ K/sec by melt extraction and the rapidly solidified alloy is then subjected to a treatment with organic or inorganic bases.

3. Raney nickel catalysts obtainable by the process according to claim 1.

4. Raney nickel catalysts obtainable by the process according to claim 2.

5. Raney nickel catalysts according to claim 3 or 4, **characterised in that** they contain as further glass-forming elements boron, silicon, carbon and/or phosphorus in an amount of 0 to 50 wt.%.

6. Use of a Raney nickel catalyst according to one of claims 3 to 5 for the hydrogenation of organic compounds.

7. Use according to claim 6 for the hydrogenation of aromatic nitro compounds.

8. Use according to claim 6 for the hydrogenation of 2,4-/2,6-dinitrotoluene mixtures.

## Revendications

1. Procédé de préparation de catalyseurs au nickel de Raney, **caractérisé en ce qu'**on laisse refroidir la fusion d'un alliage constitué de 50 à 95% en poids d'aluminium, 10 à 50% en poids de nickel, 0 à 20% en poids de fer, 0 à 15% en poids de cérium, un mischmétal de cérium, du vanadium, du niobium, du tantale, du chrome, du molybdène ou du manganèse, ainsi que le cas échéant également d'autres éléments vitrifiants présentant une vitesse de refroidissement > 10⁴ K/s par pression de la fusion d'alliage sur une roue de refroidissement en rotation ou dans l'espace entre deux roues de refroidissement en rotation tournant en sens inverse, puis on soumet l'alliage durcissant rapidement à un traitement avec des bases organiques ou inorganiques.

2. Procédé de préparation de catalyseurs au nickel de Raney, **caractérisé en ce qu'**on laisse refroidir la fusion d'un alliage constitué de 50 à 95% en poids d'aluminium, 10 à 50% en poids de nickel, 0 à 20% en poids de fer, 0 à 15% en poids de cérium, un mischmétal de cérium, du vanadium, du niobium, du tantale, du chrome, du molybdène ou du manganèse, ainsi que le cas échéant également d'autres éléments vitrifiants présentant une vitesse de refroidissement > 10⁴ K/s par extraction de la fusion, puis on soumet l'alliage durcissant rapidement à un traitement avec des bases organiques ou inorganiques.

3. Catalyseurs au nickel de Raney qu'on peut obtenir selon le procédé de la revendication 1.

4. Catalyseurs au nickel de Raney qu'on peut obtenir selon le procédé de la revendication 2.

5. Catalyseurs au nickel de Raney selon la revendication 3 ou 4, **caractérisés en ce qu'**ils contiennent comme autres éléments vitrifiants du bore, du silicium, du carbone et/ou du phosphore dans une quantité de 0 à 50% en poids.

6. Utilisation d'un catalyseur au nickel de Raney selon l'une des revendications 3 à 5, pour l'hydrogénation de composés organiques.

7. Utilisation selon la revendication 6 pour l'hydrogénation de composés nitro aromatiques.

8. Utilisation selon la revendication 6 pour l'hydrogénation de mélanges de 2,4-/2,6-dinitrotoluène.
